# EUROPEAN PATENT APPLICATION

(11) **EP 2 669 681 A1**
(43) Date of publication of application: **04.12.2013**
(21) Application number: 13382198.3
(22) Date of filing: 29.05.2013
(51) Int. Cl.: G01N 33/53, G01N 33/558, G01N 33/569

(54) **Device for the rapid diagnosis of diseases caused by Clostridium difficile in stool samples**

(30) Priority: 29.05.2012 ES 201230810
(71) Applicant: Certest Biotec, S.L., 50840 San Mateo De Gallego (Zaragoza) (ES)
(72) Inventor: Velasco Michelena, Beatriz, 50840 San Mateo de Gallego (Zaragoza) (ES); Cartagena Lirola, Hugo Fernando, 50840 San Mateo de Gallego (Zaragoza) (ES); Esquivias Ruiz-Dana, Paula, 50840 San Mateo de Gallego (Zaragoza) (ES)
(74) Representative: Carpintero Lopez, Francisco

(57) **Abstract**

The present invention relates to an immunochromatographic device for the rapid diagnosis of diseases caused by *Clostridium difficile* which, by means of applying the stool sample dispersed in a diluent, allows the simultaneous and differentiated detection of fecal *Clostridium difficile* glutamate dehydrogenase and lactoferrin and/or calprotectin by means of forming conjugates with colored particles and capturing them in a porous membrane.

## Description

### Field of the Invention

The present invention is comprised in the area of biotechnology, and specifically in the diagnosis of gastrointestinal diseases caused by *Clostridium difficile.* The object of the invention is a device that allows simple diagnosis of the diseases caused by *Clostridium difficile* (CD) by means of detecting *Clostridium difficile* glutamate dehydrogenase (GDH) and human lactoferrin (hLf) and/or human calprotectin (hCp).

### State of the Art

*Clostridium difficile* is a gram-positive anaerobic bacillus that forms spores which allow it to survive in adverse conditions (dryness, heat, salinity,...). *Clostridium difficile* is one of the enteric pathogens most frequently identified in hospitalized patients.

*Clostridium difficile* infection is the main cause of diarrhea, colitis and pseudomembranous colitis associated with taking antibiotics. There are strains that produce toxins (A and/or B) and other strains that do not produce toxins. Only the toxigenic strains cause diarrhea.

The ways of diagnosing an infection caused by *Clostridium difficile* are:
A. Culturing and isolating from stool samples. This is done in anaerobic conditions and is very slow and costly. It is not very sensitive, but it allows performing antibiotic susceptibility testing for a possible treatment.
B. Cell culture cytotoxicity. This method is considered the reference method. It has a specificity and sensitivity close to 100%. However, it is a slow (more than two days) and costly (cell culture) technique requiring highly specialized personnel.
C. Polymerase chain reaction (PCR). It is a specific and sensitive. method that further allows distinguishing different variants. It is expensive, requires specialized personnel and sophisticated equipment.
D. Enzyme immunoassays (ELISA). These assays detect the presence of toxins A and B together or separately. It is easier and faster (2 hours) than the cell culture cytotoxicity and PCR but has low sensitivity (45-95%) because the concentration of toxins in stool is often extremely low. To overcome the problem of low sensitivity of the immunoassays for detecting toxins, the use thereof combined with the detection of *Clostridium difficile* glutamate dehydrogenase (GDH), which has suitable sensitivity and specificity even though it is not capable of distinguishing between pathogenic (toxigenic) strains and non-pathogenic (non-toxigenic) strains (EP 1019724), has been proposed. However this method presents low sensitivity for pathogenic (toxin-producing) strains.
E. lmmunochromatography. Immunochromatographic (IC) tests are fast, inexpensive and easy to conduct. There are several types of tests on the market: tests detecting toxins such as *C. diff* QUICK CHECK (Techlab, Inc., Blacksburg, VA, USA) that present the same lack of sensitivity as ELISA due to the often extremely low concentration of toxins A and/or B in stool; and tests detecting *Clostridium difficile* glutamate dehydrogenase (GDH), such as the ImmunoCard® *C. difficile* GDH Test (Meridian Bioscience, Cincinnati, OH, USA). These tests are very sensitive and specific but are unable to distinguish between pathogenic (toxigenic) strains and non-pathogenic (non-toxigenic) strains.

To overcome the problem of low sensitivity of immunoassays for detecting toxins, some authors (Wren, MW et al. in Br J Biomed Sci. 2009; 66 (1); 1-5) have proposed determining fecal lactoferrin as an aid in diagnosing diseases caused by *Clostridium difficile.*

The fecal lactoferrin (hLf) is a marker of the presence of leukocytes normally associated with inflammatory processes. European patent EP0641441 describes an *in vitro* test for the determination of leukocytes in stool samples using an immunoassay with anti- lactoferrin antibodies.

Like lactoferrin, calprotectin (hCp) is an inflammatory marker. It is released by leukocytes activated by inflammatory or infectious processes. Patent US5455160 describes an immunological method for detecting fecal human calprotectin and the use thereof for screening for some bowel diseases, such as inflammatory bowel disease and the colorectal cancer.

Patent document WO2012052586 (A1) describes a rapid immunoassay for the simultaneous and differentiated detection of fecal human calprotectin and human lactoferrin and the application in the diagnosis of some bowel diseases in which inflammatory processes are involved, such as inflammatory bowel disease and the colorectal cancer.

In the foregoing description, the immunoassays for GDH and calprotectin and/or lactoferrin are different and usually based on different techniques. A specific immunoassay for GDH and another specific immunoassay for calprotectin and/or lactoferrin are performed, which means that different samples must be prepared in different diluents and different assays must be performed, which involves:
a. More time spent for performing the immunoassays.
b. A larger number of manipulations with the inherent risk of error.
c. The results obtained for the two parameters are not directly correlated because they come from two different samples or techniques.

The object of the present invention is a rapid diagnostic device of the immunochromatographic (IC) type for the simultaneous and differentiated determination of GDH, calprotectin and/or lactoferrin in stool samples having the following advantages:
a. They are simultaneously assayed, so the assay time and the number of manipulations are reduced, thereby reducing the risk of error. The simultaneous determination of the markers means that the test is more sensitive.
b. The determination is differentiated, i.e., the presence of calprotectin, lactoferrin and GDH is viewed in different lines. The differentiated determination of the markers means that the test can be more specific.
c. Specific vials that allow quantitative sampling and dispersing the samples in the diluent in an easy and hygienic manner are used.

### Brief Description

The diagnostic method of the present invention consists of: taking a representative portion of the stool sample, dispersing it in a specific diluent, applying this dispersion of the sample in a specific immunochromatographic device, forming complexes between the antibodies of the device and the antigens of the sample, and viewing these complexes in the membrane of the device.

The immunochromatographic device used is a single-use device. No type of instruments are required for carrying out the method or interpreting the result. The sample used is stool.

The preparation time of the sample is about two minutes and the test development time is from 5 to 10 minutes, which is between 10 and 20 times less than ELISA or PCR assays, which are much more complex and require laboratory instruments.

Given its simplicity, the test can be conducted in a doctor's office, and with the suitable instructions, even the patient himself/herself can conduct the test.

### Description of the Drawings

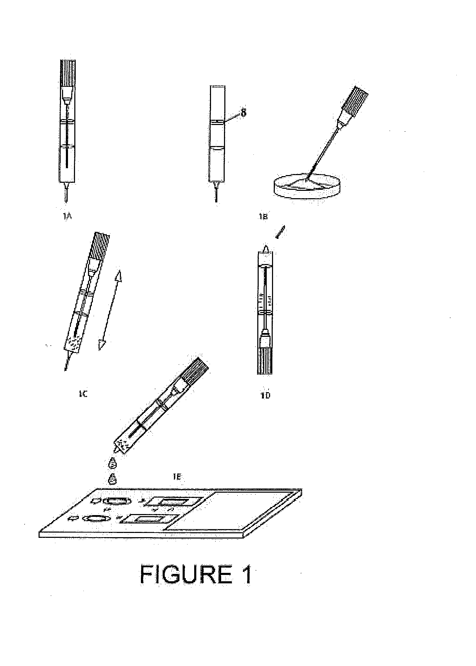

Figure 1 shows a diagram of the steps of the diagnostic method. The specific device or vial for quantitative stool sampling (Figure 1A) is a plastic vial with a capacity of about 2-3 mL in which 1 mL of diluent has previously been dispensed, with a screw-on cap having a rod the end of which, designed for this purpose, allows the quantitative collection of a solid or semi-solid stool sample (Figure 1B) when, after passing through the constricting ring (8), it is introduced in the vial. After this, the vial is closed and vigorously shaken (Figure 1C) until complete dispersion of the sample. Finally, the opposite part of the device (Figure 1D) is broken and drops are added on the sample application site of the immunochromatographic device (Figure 1 E). If quantitative sampling is not required, a vial having similar features but without the constricting ring (8) is used.
Figure 2 shows views of the diagnostic device.
Figure 2A shows a view of the immunochromatographic strip for the detection of GDH. Said strip includes the following elements: an absorbent material (1), a porous membrane (2) with a control line (3), a line for the detection of GDH (4), a sample application site (7) and a site where the antibody-marker conjugate (6) has been deposited.
Figure 2B shows a top view of the open diagnostic device, where the two immunochromatographic strips placed parallel to one another on the lower part of the device are observed.
Figure 2C shows a top view of the diagnostic device, where the result windows and the sample application points (S) on the upper face of the device are observed. A and B indicate the position of each of the immunochromatographic strips. C and T indicate the position of the lines of the test.

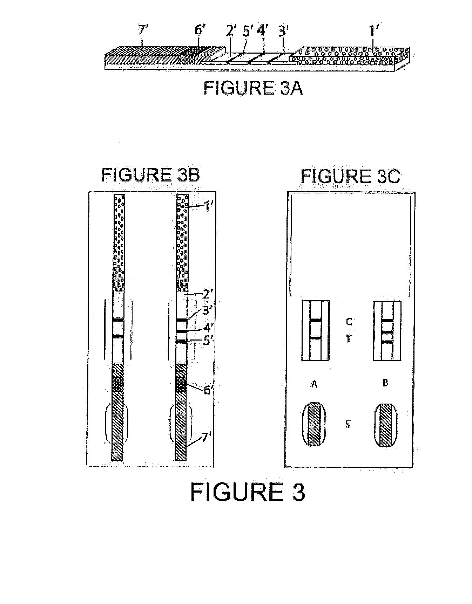

Figure 3 shows views of the diagnostic device.
Figure 3A shows a view of the immunochromatographic strip for the detection of lactoferrin and calprotectin. Said strip includes the following elements: an absorbent material (1'), a porous membrane (2') with a control line (3'), a calprotectin detection line (4') and another lactoferrin detection line (5'), a sample application site (7') and a site where the antibody-marker conjugate (6') has been deposited.
Figure 3B shows a top view of the open diagnostic device where the two immunochromatographic strips placed parallel to one another on the lower part of the device are observed.
Figure 3C shows a top view of the diagnostic device, where the result windows and the sample application points (S) on the upper face of the device are observed.

### Detailed Description

The present invention relates to a device with two immunochromatographic strips: a strip for the sensitive detection of GDH and another strip for the quantitative detection of lactoferrin and/or calprotectin. There are three possibilities: (A) an immunochromatographic GDH test strip and another immunochromatographic lactoferrin test strip; (B) an immunochromatographic GDH test strip and another immunochromatographic calprotectin test strip; (C) an immunochromatographic GDH test strip and another immunochromatographic test strip for the simultaneous detection of lactoferrin and calprotectin.

For GDH detection, a single anti-GDH monoclonal antibody is used. This monoclonal antibody (GD10) has been developed using highly purified GDH. The monoclonal antibody obtained has a high affinity and specificity for the complex formed by GDH subunits. For calprotectin detection, a single anti-calprotectin monoclonal antibody is used. This monoclonal antibody (CA1) has been developed using highly purified calprotectin. Like in the previous case, the monoclonal antibody obtained has a high affinity and specificity for the complex formed by the two calprotectin subunits, whereas it barely binds to each of the subunits separately. Finally, for lactoferrin detection, rabbit polyclonal anti-human lactoferrin antibodies are used. These antibodies were obtained by immunizing rabbits with purified human lactoferrin and were subsequently purified by affinity chromatography against the same antigen (purified human lactoferrin).

With the described antibodies, conjugates are prepared separately with colored nanoparticles. These nanoparticles are deposited in each of the strips of the assay device and act as the mobile phase. The same antibodies are immobilized separately on the membrane which acts as the stationary phase where the immunological capture of either *Clostridium difficile* GDH or of human calprotectin and/or lactoferrin takes place. The membranes and the materials with the conjugates are assembled with the aid of other materials on a plastic support and the assembly is cut in the form of a strip. These strips are introduced in the diagnostic device.

A cutoff value for the lactoferrin and calprotectin concentration is used to improve the diagnostic usefulness of the device of the invention. This cutoff value has been established at 10 µg hLf/g and 50 µg hCp/g of stool. The cutoff value is not necessary for GDH detection because since it is an antigen of an infectious agent, maximum sensitivity is required.

Quantitative sampling is necessary to perform the assay with a cutoff value. A calibrated vial is used to facilitate quantitative sampling. This vial has a rod attached to the cap of the vial which has notches such that as it passes through the constricting ring incorporated in the body of the vial, it introduces only a known amount of sample, which in this case is 10 mg (or 10 µL in the event of a liquid sample).

To perform the assay, it is necessary to prepare two vials from the same stool sample. To determine lactoferrin and/or calprotectin, the stool sample is resuspended in the sample dispersion diluent at an approximate proportion of 1/100. If the sample is a liquid sample, 10 µL of sample are mixed with 1 mL of diluent. If the sample is a solid sample, 10 mg of sample are separated with the aid of a spatula and are dispersed in 1 mL of diluent. The use of the sampling vial in which the sample is introduced with the aid of a rod, after which the vial is closed with the screw-on cap and vigorously shaken to subsequently break the upper part of the cap and add 4-5 drops on the sample application point of the immunochromatographic device, is very convenient. The design of the rod is such that upon passing through the constricting opening of the vial, it allows introducing a predetermined amount of stool sample, in this case 10 mg.

To determine GDH, the stool sample is resuspended in the sample dispersion diluent at an approximate proportion of 1/10. If the sample is a liquid sample, 100 µL of sample are mixed with 1 mL of diluent. If the sample is a solid sample, 100 mg of sample are separated with the aid of a spatula and are dispersed in 1 mL of diluent. The use of the sampling vial in which the sample is introduced with the aid of a rod, after which it is closed with the screw-on cap and vigorously shaken to subsequently break the upper part of the cap and add 4-5 drops on the sample application point of the immunochromatographic device, is very convenient. Manipulation of the sample is therefore a simple, rapid and hygienic method.

For greater ease and convenience, the diagnostic device, the diluent and the vials can be grouped together with the instructions for use in the form of a kit for performing one or more diagnostic tests.

### Analytical interpretation of the test results

After applying the sample in the device, it is necessary to wait 10 minutes to view the lines and interpret the result. The test is considered negative if only the control lines appear in the result windows. The test is considered positive for GDH if, in addition to the control line, a line appears in the position of the strip for GDH. The test is considered positive for calprotectin if, in addition to the control line, a line appears in the corresponding strip in the position for calprotectin (for example, red); the test is considered positive for lactoferrin if, in addition to the control line, a line appears in the strip in the position for lactoferrin (for example, blue); and the test is considered positive for both (hLf and hCp) if three lines appear (for example, green, red and blue).

### Diagnostic interpretation of the test results

A negative result for GDH, hLf and hCp indicates that there is no *Clostridium difficile* infection. Positive for GDH and negative for hLf and hCp indicates that there is *Clostridium difficile* colonization, but of a non-pathogenic (non-toxigenic) strain. Finally, a positive result for GDH and a positive result for hLf or hCp indicates an inflammatory process caused by a *Clostridium difficile* infection.

### Embodiments

### Example 1

### Obtaining Clostridium difficile GDH: Cloning the Clostridium difficile gluD gene

The *Clostridium difficile gluD* (Glutamate Dehydrogenase, GenBank accession number M65250) gene was cloned into the *Nco*I/*Xho*I sites of the pET-30b(+)plasmid (Merck KGaA, Darmstadt, Germany) using genomic DNA from the standard toxigenic VP1 10463 strain (ATCC 43255) as the template for the PCR amplification of said gene.

The genomic DNA was extracted from the VP1 10463 strain by means of the QlAamp DNA Minikit (Qiagen GmbH, Hilden, Germany) following the manufacturer's instructions.

The *gluD* gene (1,282 bps) was amplified using DNA Polymerase TaKaRa LA Taq (Takara Bio Inc., Shiga, Japan) and primers *gluD*1 (AGGACCATGGCTTCAGGAAAAGATGTAAATGTC) and *gluD2* (CAGCCTCGAGTTAGTACCATCCTCTTAATTTCATAGC). Deoxynucleotide triphosphates (2.5 mM of each), primers (0.2 µM of each), 5 µl of 10x LA PCR Buffer (Mg²⁺), 2 units of DNA polymerase TaKaRa LA Taq, 50 ng of genomic DNA and purified water were added to the PCR mixture to a final volume of 50 µL. The DNA was amplified by means of an initial denaturation of 1 minute at 94°C, 30 denaturing (10 seconds at 98°C), annealing (30 seconds at 58°C) and elongation (30 seconds at 72°C) cycles and a final elongation cycle of 10 minutes at 72°C.

The PCR product was viewed by 1% agarose gel electrophoresis prepared in TBE buffer (89 mM Tris, 89 mM boric acid, 2 mM EDTA, pH 8.4) and stained with 1x GelRed (Biotium Inc., Hayward, CA, USA). The size of the DNA bands was estimated with the aid of the molecular weight marker called Perfect DNA Markers (0.1-12 Kb) (Merck KGaA, Darmstadt, Germany).

The resulting DNA fragment was isolated and purified by means of Ultra Clean PCR Clean-Up columns (Mo BioLaboratories, Inc., Carlsbad, CA, USA) and cloned into the pET-30b(+) plasmid after digestion of both the purified PCR product and of the plasmid with restriction enzymes *Nco*I and *Xho*I (Takara Bio Inc., Shiga, Japan) and subsequent ligation with the enzyme T4DNA Ligase (Takara Bio Inc, Shiga, Japan).

Competent DH5α *Escherichia coli* cells (Invitrogen Corporation, Carlsbad, CA, USA) were transformed with the resulting plasmid. The positive clones were selected in LB + 50 µg/mL kanamycin plates. The presence of the pET-30b(+) plasmid + *gluD* in the cells was checked by means of PCR and digestion with restriction enzymes.

### Obtaining Clostridium difficile GDH: Expression and purification of GDH protein in E. coli

The glutamate dehydrogenase (GDH) protein was expressed in competent BL21 (DE3) pLysS *E coli* cells (Promega, Southampton, United Kingdom). These cells were transformed with the pET-30b(+) plasmid + *gluD* and the positive clones were selected in LB + 50 µg/mL kanamycin + 25 µg/mL chloramphenicol plates.

To induce the expression of GDH, one of these colonies was inoculated in 80 ml of LB + 50 µg/mL kanamycin + 25 µg/mL chloramphenicol and was cultured overnight at 37°C under stirring (225 rpm). The next day the culture was diluted 20 fold in a final volume of 1,200 mL of LB + 50 µg/mL kanamycin + 25 µg/mL chloramphenicol and was incubated maintaining the same temperature and stirring conditions until the culture reached an OD of 0.5 at 600 nm. Then IPTG at a concentration of 1 mM was added and the cells were cultured for 6 hours at 37°C and under stirring at 225 rpm to induce the expression of GDH. The cells were collected by means of centrifugation at 11,000 rpm at room temperature for 5 minutes.

To obtain the protein extract, the cell pellet was resuspended in a volume of 5 ml of Lysis Buffer (0.2 mg/mL Lysozyme from chicken egg white (Sigma-Aldrich GmbH, Steinheim, Germany), 20 µg/mL Deoxyribonuclease I from bovine pancreas (Sigma-Aldrich GmbH, Steinheim, Germany), 20 µg/mL RNAse A (Applichem GmbH, Darmstadt, Germany), 1 mM MgCl₂ (Sigma-Aldrich GmbH, Steinheim, Germany), 1 mM PMSF (Sigma-Aldrich GmbH, Steinheim, Germany), 1 entire ULTRA Tablet Mini EDTA-free, EASYpack (Roche Diagnostics GmbH, Mannheim, Germany), Binding Buffer (20 mM sodium phosphate, 0.5 M NaCl, 50 mM imidazole, pH 7.4) per gram of cells and was incubated for an hour at 4°C.

After this time elapsed, the samples were sonicated 5 times for 30 seconds with 1 minute intervals in ice at an intensity of 7 in a Branson Sonifier 250 (Branson Ultrasonics Co., Danbury, CT, USA) and were centrifuged at 11,000 rpm for 30 minutes at 4°C. The supernatant was filtered with a 0.2 µm membrane (Pall Corporation, Ann Arbor, Ml, USA).

The recombinant protein was purified by immobilized-metal affinity chromatography (IMAC) using a HisTrap HP column (GE Healthcare Bio-Sciences AB, Uppsala, Sweden). The column was pre-equilibrated beforehand with 10 volumes of binding buffer (20 mM sodium phosphate, 0.5 M NaCl, 50 mM imidazole, pH 7.4) and then the filtered protein extract was applied to the column at a flow rate of 5 mL/minute. Then the column was washed with 10 volumes of binding buffer, maintaining the flow rate of 5 mL/minute. The recombinant protein bound to the column was eluted at a flow rate of 2.5 mL/minute with elution buffer (20 mM sodium phosphate, 0.5 M NaCl, 500 mM imidazole, pH 7.4) and fractions were collected. The degree of purity was determined by means of polyacrylamide gel electrophoresis under denaturing conditions (SDS-PAGE). The histidine tail was removed by enzymatic digestion with enterokinase (4µL/mg of protein, New England Biolabs, lpswhich, MA, United Kingdom) for 16 hours at 23°C and subsequently passed through a HisTrap HP column (GE Healthcare Bio-Sciences AB, Uppsala, Sweden).

### Example 2

### Preparation and purification of anti-GDH monoclonal antibodies

GD10 monoclonal antibody is purified from the hybridome culture bearing the same name. The hybridoma producing GD10 monoclonal antibody was obtained by means of protocols known according to the method of fusing cells and selecting the obtained clones, which was initially described by Kohler and Milstein in 1975 (Köhler G, Milstein C. Continuous cultures of fused cells secreting antibody of predefined specificity. Nature. 1975 Aug 7;256 (5517):495-7).

The method is as follows. BALB/c mice are immunized with purified *Clostridium difficile* GDH. The lymphocytes of the immunized mice are fused with SP20 line myeloma cells and the obtained hybridomas are selected by means of ELISA techniques as follows: the wells of a 96-well microtiter plate are covered with rabbit polyclonal anti-GDH antibody in 100 mM carbonate buffer with pH 9 at 37°C for 2 hours. The purified GDH is added to these wells. After washing, the different hybridoma culture samples are added, and the presence of the anti-GDH antibody is shown using a peroxidase-conjugated anti-mIgG and the corresponding substrate.

The hybridomas with the highest affinity and best specificity are selected. Among them, the most productive hybridomas and those which offer the best features in thermal stress tests and antigen reaction speed are selected.

The selected GD10 hybridoma is cultured in RPMI-HT medium for several days at 37°C with 5% CO₂. The antibody is purified from the culture medium by protein A affinity chromatography according to the column manufacturer's instructions (GE Healthcare) after which it is dialyzed in PBS pH 7.4.

### Example 3:

### Preparation of the anti-GDH conjugates

A GD10 antibody conjugate is prepared with polystyrene microparticles. Colored particles with carboxyl groups on their surface with a nominal diameter of 200 nm are used (K1 020 Estapor®, Merck, Darmstadt, Germany). 1 mL of particles at 10% are washed by centrifugation and resuspension in 10 mM MES buffer (2-(N-morpholino)ethanesulfonic acid) pH 6 and EDC (1-ethyl-3-(3-dimethylaminopropyl)-carbodiimide) is added until reaching a concentration of 5 mM. It is incubated for 1 hour at 37°C and the excess reagent is removed by centrifugation. The particles thus activated are resuspended in 10 mM MES pH 6 and the anti-GDH monoclonal antibody is added until reaching a surface concentration of 2 mg/m², after which they are incubated for 18 hours at 4°C and subsequently washed in 0.1 % Tween 20.

The conjugates with particles for the control line are obtained in the same way but using streptavidin (S4762, Sigma-Aldrich) at a final surface concentration of 1 mg/m² instead of the antibody.

### Example 4

### Preparation of GDH test strip

A mixture of the conjugates with particles with anti-GDH and streptavidin described at concentrations of 0.05% and 0.03%, respectively, are diluted in a solution containing 10% sucrose, 2% bovine casein, 2% bovine albumin, 1% PEG-6000 and 2% Tween-20 in TRIS buffer (tris(hydroxymethyl)aminomethane) pH 8.3. This solution is deposited at a ratio of 13 µL/cm in a wound material consisting of non-woven polyester fibers 29 mm wide which is dried in a 45 degree airstream after deposition (5 minutes) and for 24 hours in a chamber at 30°C and 15% relative humidity.

The anti-GDH and anti-streptavidin antibodies (for the control line) are dialyzed in PBS, taken to a concentration of 1 mg/mL and linearly deposited separately and in a parallel manner on a laminated nitrocellulose membrane (Hi-Flow Plus by Millipore) 25 mm wide and with a pore size between 10 and 30 micrometers at a ratio of 1 µL/cm, after which it is dried in a 45 degree airstream after deposition (2 minutes) and dried immediately after for 24 hours in a chamber at 30°C and 15% relative humidity.

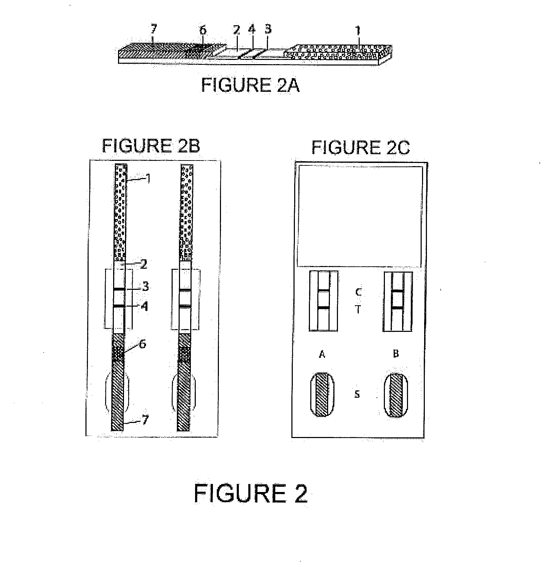

The material with the conjugate, the membrane and the absorbent material are assembled as indicated in Figure 2A on a plastic support with an adhesive sheet and the strips are cut transversely with respect to their assembly to a width of 4 mm.

### Example 5:

### Preparation of lactoferrin and calprotectin test strip

The lactoferrin and calprotectin test strip is prepared similarly to that described in patent document WO2012052586 (A1). The test strip for detecting only lactoferrin is prepared in the same way but without using the anti-hCp antibody in the membrane and without depositing the anti-calprotectin colloid in the absorbent material. The test strip for detecting only calprotectin is prepared in the same way but without the anti-hLf antibody in the membrane and without the anti-lactoferrin colloid in the absorbent material.

### Assembly of the device

The two IC strips are arranged inside a casing made of a plastic material that has been designed such that it has one housing for each of the strips, two different windows for adding samples and two other windows for viewing the results. The plastic casing can be marked or printed on for the correct indication of the position of each strip for the user.

A solution for dispersing the stool samples is prepared, consisting of an aqueous solution of 300 mM sodium chloride, 1% Triton X-100, 100 µg/mL non-specific mouse IgG antibodies and 200 µg/mL non-specific rabbit IgG antibodies in a 200 mM TRIS-HCI buffer at pH 8.5. This preparation is dispensed in vials for sampling at a ratio of 1 mL/vial.

### Example 6

### GDH detection

1/2 serial dilutions of GDH were prepared in the sample dispersion diluent described in the preceding section. 100 µL of these dilutions are applied to the immunochromatographic devices of the invention and the assay is left to develop for 10 minutes at room temperature (25°C), after which the result is interpreted by means of visual examination of the appearance of the test line. The same operation is performed but diluting GDH in a pool of about 1/10 stool samples resuspended in the same buffer. The results are shown in Table 1.

**Table 1**

| ng GDH/mL | In diluent | In stool |
|---|---|---|
| 100 | + | + |
| 50 | + | + |
| 25 | + | + |
| 12.5 | + | + |
| 6.25 | + | + |
| 3.125 | + | + |
| 1.56 | + | + |
| 0.78 | + | + |
| 0.39 | - | - |
| 0 | - | - |

### Detection of fecal calprotectin and lactoferrin

In order for the device of the invention to have a suitable diagnostic usefulness, it is necessary to use cutoff values, i.e., the concentration of the analyte above which the test is positive and below which the test is negative. In the case of calprotectin, the cutoff value is 50 µg/g of stool and for lactoferrin it is 10 µg hLf/g of stool.

Due to the existence of the cutoff value, it is necessary to take the stool sample in a quantifiable manner. A specific vial for taking stool samples containing a rod with notches for the sample and a constricting part for eliminating excess sample is used to that end, such that the system has been calibrated so that exactly 10 mg of stool sample are introduced in the vial. On the other hand and given that the vial contains 1 mL of diluent, sampling is performed quantitatively.

### Calprotectin

1/2 serial dilutions of calprotectin were prepared in the sample dispersion diluent described in the preceding section. 100 µL of these dilutions are applied to the immunochromatographic devices of the invention and the assay is left to develop for 10 minutes at room temperature (25°C), after which the result is interpreted by means of visual examination of the appearance of the test line. The same operation is performed but diluting the calprotectin in a pool of about 1/100 stool samples resuspended in the same buffer. The results are shown in Table 2.

**Table 2**

| ng hCp/mL | In diluent | In stool | µg hCp/g stool |
|---|---|---|---|
| 16000 | + | + | 1600 |
| 8000 | + | + | 800 |
| 4000 | + | + | 400 |
| 2000 | + | + | 200 |
| 1000 | + | + | 100 |
| 500 | + | + | 50 |
| 250 | - | - | 25 |
| 125 | - | - | 12.5 |
| 0 | - | - | 0 |

### Lactoferrin:

1/2 serial dilutions of lactoferrin were prepared in the sample dispersion diluent described in the preceding section. 100 µL of these dilutions are applied to the immunochromatographic devices of the invention and the assay is left to develop for 10 minutes at room temperature (25°C), after which the result is interpreted by means of visual examination of the appearance of the test line. The same operation is performed but diluting lactoferrin in a pool of about 1/100 stool samples in the same buffer. The results are shown in Table 3.

**Table 3**

| ng hLf/mL | In diluent | In stool | µg hLf/g stool |
|---|---|---|---|
| 3200 | + | + | 320 |
| 1600 | + | + | 160 |
| 800 | + | + | 80 |
| 400 | + | + | 40 |
| 200 | + | + | 20 |
| 100 | + | + | 10 |
| 50 | - | - | 5 |
| 25 | - | - | 2.5 |
| 0 | - | - | 0 |

A stool sample containing a GDH concentration equal to or greater than 0.8 ng/mL, a fecal human calprotectin concentration greater than or equal to 50 µg/g stool and a lactoferrin concentration equal to or greater than 10 µg/g in stool give positive results using the test of the present invention.

## Claims

1. Immunochromatographic device for the diagnosis of diseases caused by *Clostridium difficile* in stool samples, **characterized in that** it comprises:
a. An immunochromatographic strip for the detection of *Clostridium difficile* glutamate dehydrogenase.
b. An immunochromatographic strip for the differentiated detection of lactoferrin and calprotectin.

2. Device according to claim 1, wherein only lactoferrin is detected in addition to glutamate dehydrogenase.

3. Device according to claim 1, wherein only calprotectin is detected in addition to glutamate dehydrogenase.

4. Method for diagnosing diseases caused by *Clostridium difficile,* **characterized in that** it comprises:
a. Obtaining a stool sample,
b. Dispersing the fecal material of the sample in a buffer or diluent,
c. Applying this dispersion in the sample windows of the device of claims 1-3,
d. Interpreting the result according to the appearance of colored lines in the result windows.

5. Method for diagnosing diseases caused by *Clostridium difficile* according to claim 4, **characterized in that** it comprises:
a. Dispersing the fecal material of the sample in a vial which allows detecting *Clostridium difficile* glutamate dehydrogenase at maximum sensitivity,
b. Dispersing the fecal material of the sample in a vial for quantitative sampling for detecting lactoferrin and/or calprotectin with a defined detection threshold.

6. Kit for performing the diagnosis of diseases caused by *Clostridium difficile,* **characterized in that** it comprises:
a. An immunochromatographic device according to claims 1-3,
b. One or several diluents suitable for suspension and dispersion of the fecal material,
c. two vials for sampling and the manipulation of such samples.
